(19) Europäisches Patentamt
European Patent Office
Office européen des brevets

(11) Veröffentlichungsnummer: **0 038 343**
**B1**

# (12) EUROPÄISCHE PATENTSCHRIFT

(45) Veröffentlichungstag der Patentschrift: **27.12.84**

(51) Int. Cl.³: **C 07 C 59/90**, C 07 C 59/88, C 07 C 59/84, C 07 C 51/09, C 07 C 51/41, C 07 C 51/02, C 07 C 51/38, A 61 K 31/19, C 07 C 69/738, C 07 C 69/65, C 07 C 143/68

(21) Anmeldenummer: **80902059.7**

(22) Anmeldetag: **31.10.80**

(86) Internationale Anmeldenummer: **PCT/EP 80/00123**

(87) Internationale Veröffentlichungsnummer: **WO 81/01285 (14.05.81 Gazette 81/12)**

## (54) SUBSTITUIERTE OXOCARBONSÄUREN, VERFAHREN ZU IHRER HERSTELLUNG, IHRE VERWENDUNG UND SIE ENTHALTENDE ARZNEIMITTEL.

**Verbunden mit 80106676.2/51077 (europäische Anmeldenummer/Veröffentlichungsnummer) durch Entscheidung vom 30.07.82.**

(30) Priorität: **31.10.79 CH 9785/79**

(43) Veröffentlichungstag der Anmeldung: **28.10.81 Patentblatt 81/43**

(45) Bekanntmachung des Hinweises auf die Patenterteilung: **27.12.84 Patentblatt 84/52**

(84) Benannte Vertragsstaaten: **AT BE CH DE FR GB IT LI LU NL SE**

(56) Entgegenhaltungen:
**DE - A - 1 810 026**

**Journal of the American Chemical Society, volume 58, no. 11 November 1936 L.F. Fieser et al. "The Synthesis of phenanthrene and hydrophenanthrene derivatives V", paragraph 2320-2321 cited in the application**
**Chem. Abstracts, vol. 55, 25874g**
**Chem. Abstracts, vol. 51, 4332**

(73) Patentinhaber: **Byk Gulden Lomberg Chemische Fabrik GmbH, Byk-Gulden-Strasse 2, D-7750 Konstanz (DE)**

(72) Erfinder: **KRAAS, Ekkehard, Küsterkoppel 15, D-2401 Krummesse (DE)**
Erfinder: **LUDWIG, Gerhard, Meisenweg 6, D-7750 Konstanz 16 (DE)**
Erfinder: **RAPP, Erich, Pfarrer-Braunstrasse 6, D-7760 Radolfzell (DE)**
Erfinder: **WOLF, Horst, Brandesstrasse 29, D-7750 Konstanz (DE)**

**EP 0 038 343 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft substituierte Oxocarbonsäuren, Verfahren zu ihrer Herstellung, ihre Verwendung und sie enthaltende Arzneimittel.

In der deutschen Offenlegungsschrift DE-OS 2 365 755 werden α-Ketosäureester beschrieben, die als Zwischenprodukte für die Herstellung von Aminosäuren oder heterocyclischen Verbindungen dienen können. Aus der belgischen Patentschrift BE-PS 779 821 ist ein Verfahren zur Herstellung von α-Ketosäuren bekannt, wobei die Ketosäuren als Zwischenprodukte im Metabolismus oder als Vorstufen von Aminosäuren erwähnt werden. 5-(4-Methoxyphenyl)-2-oxovaleriansäure wurde von L.F. Fieser und H.L. Holmes [J. Amer. Chem. Soc. 58 (1936) 2319] hergestellt. C.A. 55, 25 874 g beschreibt die Herstellung von 5-(4-Fluorphenyl)-2-oxo-valeriansäure als Zwischenprodukt und C.A. 51, 4332 die Herstellung von 5-Phenyl-2-oxo- und 5-(4-Methylphenyl)-2-oxo-valeriansäure, eine pharmakologische Wirkung wird nicht offenbart. Bestimmte substituierte Oxocarbonsäuren wurden nun als pharmazeutische Wirkstoffe mit einer spezifischen Wirkung erfunden.

Gegenstand der Erfindung sind substituierte Oxocarbonsäuren der allgemeinen Formel I

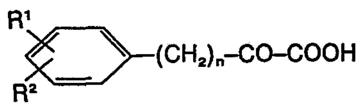
(I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat,

und ihre Salze.

Als Niederalkylgruppen kommen geradkettige oder verzweigte Alkylreste mit 1 bis 4 Kohlenstoffatomen in Betracht. Geradkettige Alkylreste sind beispielsweise der Methyl-, Ethyl-, n-Propyl- und n-Butylrest, von denen die mit 1 und 2 Kohlenstoffatomen bevorzugt sind. Verzweigte Alkylreste sind beispielsweise der Isopropyl-, Isobutyl- und sek.-Butylrest, von denen der mit 3 Kohlenstoffatomen bevorzugt ist. Als Alkylreste von Niederalkoxygruppen kommen sowohl geradkettige als auch verzweigte Niederalkylgruppen in Frage. Die Methoxygruppe ist als Niederalkoxygruppe bevorzugt.

Halogenatome sind Fluor-, Chlor- und Bromatome, von denen Fluor, insbesondere Chlor, bevorzugt sind.

Die Substituenten $R^1$ und $R^2$ stehen bevorzugt in meta- oder para-Stellung zum Ketocarbonsäurerest.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemässen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basische Aminosäuren, etc. zu Anwendung.

Beispielsweise seien die Salze von Ethylendiamin, Dimethylamin, Diethylamin, Morpholin, Piperidin, Piperazin, N-Niedrigalkylpiperazin (z.B. N-Methylpiperazin), Methylcyclohexylamin, Benzylamin, Ethanolamin, Diethanolamin, Triethanolamin, Tris-(hydroxymethyl)-aminomethan, 2-Amino-2-methylpropanol, 2-Amino-2-methyl-1,3-propandiol, Glucamin, N-Methylglucamin, Glucosamin, N-Methylglucosamin, Lysin, Ornithin, Arginin und Chinolin, vorzugsweise von Lithium, Natrium, Kalium, Magnesium, Calcium und Aluminium, genannt.

Eine Ausgestaltung der Erfindung sind substituierte Oxocarbonsäuren der allgemeinen Formel I*

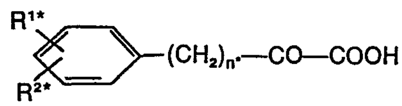
(I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom oder eine Methoxygruppe und $R^{2*}$ ein Wasserstoffatom bedeuten, wenn n* die Bedeutung 3 hat,

und ihre Salze.

Eine bevorzugte Ausgestaltung der Erfindung sind substituierte Oxocarbonsäuren der allgemeinen Formel I**

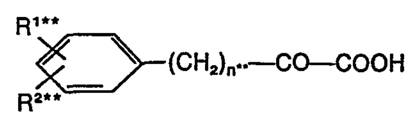
(I**),

worin $R^{1**}$ und $R^{2**}$ in meta- oder para-Stellung zum Ketocarbonsäurerest stehen und $R^{1**}$ ein Wasserstoffatom oder ein Chloratom, $R^{2**}$ ein Chloratom und n** 3 bis 5 bedeuten, und ihre pharmakologisch verträglichen Salze mit anorganischen oder organischen Basen.

Als Vertreter der erfindungsgemässen Säuren seien beispielsweise

6-(2,4-Dichlorphenyl)-2-oxocapronsäure,
2-Oxo-6-(3-trifluormethylphenyl)-capronsäure,
5-(3,4-Dichlorphenyl)-2-oxovaleriansäure,
7-(3-Chlorphenyl)-2-oxoheptansäure,
7-(4-Methoxyphenyl)-2-oxoheptansäure,
7-(4-Bromphenyl)-2-oxoheptansäure,
8-(4-n-Butoxyphenyl)-2-oxo-octansäure,
8-(3-Fluorphenyl)-2-oxo-octansäure,
2-Oxo-8-(3-trifluormethylphenyl)-octansäure,
7-(4-Methylphenyl)-2-oxo-heptansäure,

8-(4-Hydroxyphenyl)-2-oxo-octansäure,
9-(4-Chlorphenyl)-2-oxo-nonansäure,
9-(3-Trifluormethylphenyl)-2-oxo-nonansäure,
10-(2,4-Dichlorphenyl)-2-oxo-decansäure,
10-(4-Methylphenyl)-2-oxo-decansäure
genannt.

Bevorzugte Vertreter sind
2-Oxo-6-phenylcapronsäure,
6-(4-Methoxyphenyl)-2-oxo-capronsäure,
5-(4-Chlorphenyl)-2-oxo-valeriansäure
und ihre pharmakologisch verträglichen Salze.

Die erfindungsgemässen Verbindungen weisen wertvolle pharmakologische Eigenschaften auf, die sie gewerblich verwertbar machen. Sie wirken hypoglycämisch.

Aufgrund ihrer vorteilhaften Wirksamkeit sind die erfindungsgemässen substituierten Oxocarbonsäuren der allgemeinen Formel I bzw. I′ sowie der Ausgestaltungen I* und I** sowie ihre Salze zur Behandlung und Prophylaxe von Krankheiten, die auf Störungen des Glucosestoffwechsels beruhen, geeignet. Beispielsweise werden behandelt prädiabetische Zustände zur Verhinderung der Manifestation des Diabetes, manifester Diabetes, z. B. Erwachsenendiabetes, labiler Diabetes von Jugendlichen.

Gegenstand der Erfindung ist daher auch eine Methode zur Bekämpfung der angegebenen Krankheiten durch Applikation der erfindungsgemässen Verbindungen. Gegenstand der Erfindung ist ferner die Verwendung der erfindungsgemässen Verbindungen bei der Bekämpfung der angegebenen Krankheiten.

Ein weiterer Gegenstand der Erfindung sind Arzneimittel, die eine oder mehrere der substituierten Oxocarbonsäuren der allgemeinen Formel I′

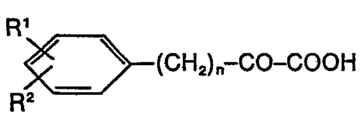

(I′),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und/oder ihre pharmakologisch verträglichen Salze mit anorganischen oder organischen Basen enthalten.

Ausgestaltungen der Arzneimittel sind solche, die substituierte Oxocarbonsäuren der Formel I′, in der $R^1$ ein Wasserstoffatom, ein Chloratom, eine Methylgruppe, eine Methoxygruppe oder eine Trifluormethylgruppe, $R^2$ ein Wasserstoffatom oder ein Chloratom und n eine ganze Zahl von 3 bis 6 bedeuten, I* oder I** oder die pharmakologisch verträglichen Salze der Säuren mit anorganischen oder organischen Basen, enthalten.

Gegenstand der Erfindung ist ausserdem die Verwendung der erfindungsgemässen Verbindungen zur Herstellung von Arzneimitteln zur Bekämpfung der angegebenen Krankheiten.

Die Arzneimittel werden nach an sich bekannten Verfahren hergestellt. Als Arzneimittel können die neuen Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, beträgt der Wirkstoffgehalt dieser Mischungen 1 bis 95, vorzugsweise 15 bis 85 Gewichtsprozent der Gesamtmischung.

In Übereinstimmung mit der Erfindung können im humanmedizinischen Bereich die Wirkstoffe in beliebiger Form, z. B. systemisch, angewandt werden unter der Voraussetzung, dass die Ausbildung bzw. Aufrechterhaltung von ausreichenden Blut- oder Gewebsspiegeln an Wirkstoffen gewährleistet ist. Das kann beispielsweise durch orale oder parenterale Gabe in geeigneten Dosen erreicht werden. Vorteilhafterweise liegt die pharmazeutische Zubereitung des Wirkstoffes in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulats, einer Lösung, einer Emulsion oder einer Suspension sein.

Unter «Einheitsdosis» im Sinne der vorliegenden Erfindung wird eine physikalisch bestimmte Einheit, die eine individuelle Menge des aktiven Bestandteiles in Kombination mit einem pharmazeutischen Trägerstoff enthält, verstanden, deren Wirkstoffgehalt einem Bruchteil oder Vielfachem einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäss der Erfindung enthalten, wenn sie in Einheitsdosen vorliegen und für die Applikation, z. B. am Menschen bestimmt sind, 2 bis 200 mg, vorteilhafterweise 10 bis 100 mg und insbesondere 20 bis 60 mg Wirkstoff.

Im allgemeinen hat es sich in der Humanmedizin als vorteilhaft erwiesen, den oder die Wirkstoffe bei oraler Gabe in einer Tagesdosis von 0,1 bis 30, vorzugsweise 0,3 bis 15, insbesondere 0,6 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von 0,05 bis 10, vorzugsweise 0,1 bis 5, insbesondere 0,3 bis 1 mg/kg Körpergewicht.

Bei einer parenteralen Behandlung, z. B. einer intravenösen oder intramusculären Applikation können ähnliche Dosierungen zur Anwendung kommen. Bei dieser Therapie werden 0,3 bis 1 mg Wirkstoff/kg Körpergewicht appliziert.

Die therapeutische Verabreichung der pharmazeutischen Zubereitung erfolgt bei Dauermedikation im allgemeinen zu festgelegten Zeitpunkten, wie 1 bis 4 mal am Tage, z.B. jeweils nach den Mahlzeiten und/oder am Abend. Bei akuten Anlässen erfolgt die Medikation zu variierendem Zeitpunkt. Unter bestimmten Gegebenheiten kann es erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikation der Wirkstoffe nimmt der Fachmann aufgrund seines Fachwissens vor.

Die pharmazeutischen Zubereitungen bestehen in der Regel aus den erfindungsgemässen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süssungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wässrige und ölige Suspensionen, Emulsionen oder Lösungen kommen.

Tabletten können inerte Verdünnungsmittel, z.B. Calciumcarbonat, Calciumphosphat, Natriumphosphat oder Xylit; Granulierungs- und Verteilungsmittel, z.B. Calciumphosphat oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass eine verzögerte Auflösung und Resorption des Arzneimittels im Gastrointestinaltrakt und damit z.B. eine bessere Verträglichkeit, Protrahierung oder eine Retardierung erreicht wird. Gelatinekapseln können den Arzneistoff vermischt mit einem festen Verdünnungsmittel, z.B. Calciumcarbonat oder Kaolin, oder einem öligen Verdünnungsmittel, z.B. Paraffinöl, enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süssungsmittel, z.B. Saccharin, Natriumcyclamat, enthalten.

Ölige Suspensionen können z.B. Paraffinöl und Verdickungsmittel, wie Bienenwachs, Hartparaffin oder Cetylalkohol, enthalten; ferner Süssungsmittel, Geschmacksmittel und Antioxidantien.

In Wasser dispergierbare Pulver und Granulate können die Arzneistoffe in Mischung mit Dispergier-, Benetzungs- und Suspendiermitteln, z.B. den obengenannten, sowie mit Süssungsmitteln, Geschmacksmitteln und Farbstoffen enthalten.

Emulsionen können z.B. Paraffinöl neben Emulgiermitteln, wie Akaziengummi, Traganthgummi, Phosphatiden, Sorbitanmonooleat, Polyoxyethylensorbitanmonooleat, und Süssungs- und Geschmacksmitteln enthalten.

Zur parenteralen Anwendung der Arzneistoffe dienen steril injizierbare wässrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglycol, enthalten können.

Der oder die Wirkstoffe können gegebenenfalls mit einem oder mehreren der angegebenen Träger- oder Zusatzstoffe auch in mikroverkapselter Form formuliert werden.

Neben den erfindungsgemässen substituierten Oxocarbonsäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u.a.), z.B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze enthalten.

Ein weiterer Gegenstand der Erfindung ist ein Verfahren zur Herstellung der substituierten Oxocarbonsäuren der allgemeinen Formel I

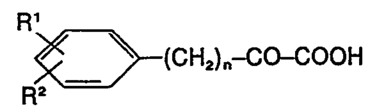 (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom oder eine Methoxygruppe und $R^2$ ein Wasserstoffatom bedeuten, wenn n die Bedeutung 3 hat,

und ihrer Salze, dadurch gekennzeichnet, dass man

a) einen Oxocarbonsäureester der allgemeinen Formel II

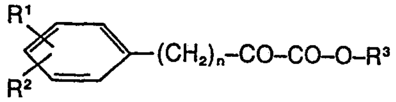
(II),

worin $R^1$, $R^2$ und n die vorstehend angegebene Bedeutung haben und $R^3$ einen Niederalkylrest bedeutet, solvolysiert und gegebenenfalls anschliessend erhaltene Säuren in die Salze oder erhaltene Salze in die Säuren überführt, oder

b) einen Diester der allgemeinen Formel III

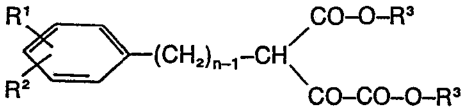
(III),

worin $R^1$, $R^2$, $R^3$ und n die vorstehend angegebene Bedeutung haben, solvolysiert und decarboxyliert und gegebenenfalls anschliessend erhaltene Säuren in die Salze oder erhaltene Salze in die Säuren überführt.

Die Verbindungen der allgemeinen Formel I werden nach an sich bekannten Verfahren hergestellt. Die Herstellung der Verbindungen I kann neben den angegebenen Verfahrensvarianten auch analog anderen in der Literatur beschriebenen Verfahren erfolgen, beispielsweise seien genannt: Die in der BE-PS 779 821 angegebenen Methoden, die Verfahren von H. Poisel [Ber. 111 (1978) 3136], R. Fischer und T. Wieland [Ber. 93 (1960) 1387], J. Anatol und A. Medète [Synthesis 1971, 538], E.E. Eliel und A.A. Hartmann [J. org. Chem. 37 (1972) 505], K. Tanaka et al. [Tetrahedron Letters 1978, 4809], K. Ogura et. al. [Tetrahedron Letters 1978, 375].

Die Solvolyse der Oxocarbonsäureester II und der Diester III erfolgt beispielsweise mit einer wässerigen oder alkoholischen (z.B. ethanolischen) Alkalimetallhydroxid- (z.B. Kaliumhydroxid-)Lösung bei Raumtemperatur, gegebenenfalls unter Zusatz eines inerten Verdünnungsmittels, wie Dioxan oder Toluol. Bevorzugt erfolgt die Solvolyse mit wässerigen Lösungen von Mineralsäuren, wie Salzsäure, Bromwasserstoffsäure, Schwefelsäure, gegebenenfalls unter Zusatz eines organischen Lösungsmittels, wie Dioxan oder Diglyme, bei Temperaturen zwischen 0 °C und der Siedetemperatur des Lösungsmittels, bevorzugt zwischen 20 und 70 °C. Die Decarboxylierung der Diester III erfolgt durch Erhitzen der jeweiligen Lösungen, gegebenenfalls gleichzeitig mit der Solvolyse.

Die Überführung der Oxocarbonsäuren der allgemeinen Formel I bzw. der Ausgestaltungen I* und I** in die Salze kann durch direkte alkalische Solvolyse, z.B. Hydrolyse, der Ester II ($R^3$ = Niederalkyl) erfolgen. Als alkalischer Reaktionspartner wird diejenige anorganische oder organische Base verwendet, deren Salze gewünscht wird. Man erhält die Salze aber auch, indem man die Säuren I mit dem stöchiometrischen Äquivalent an entsprechender Base, z.B. Natriumhydroxid oder Natriumethanolat umsetzt, oder leicht lösliche Salze durch doppelte Umsetzung in schwer lösliche Salze überführt, oder beliebige Salze in pharmakologisch verträgliche Salze überführt. Die Herstellung der freien Oxosäuren I ist gegenüber der der Salze bevorzugt.

Zur Herstellung der substituierten Oxocarbonsäuren der Ausgestaltungen I* und I** werden Oxocarbonsäureester der allgemeinen Formeln II* und II** bzw. Diester III* und III**

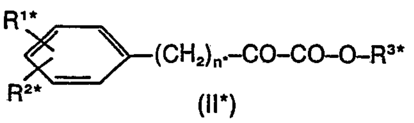
(II*)

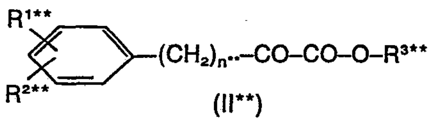
(II**)

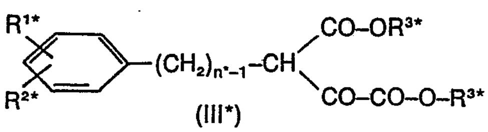
(III*)

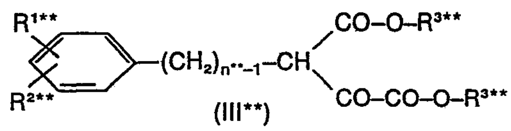
(III**)

worin $R^{1*}$, $R^{2*}$ und n* bzw. $R^{1**}$, $R^{2**}$ und n** die oben angegebene Bedeutung haben, $R^{3*}$ einen Niederalkylrest und $R^{3**}$ einen Methyl- oder Ethylrest bedeutet, eingesetzt.

Die Herstellung der Oxocarbonsäureester der allgemeinen Formeln II, II* und II** erfolgt nach an sich bekannten Verfahren, z.B. gemäss der DE-OS 2 365 755. Sie kann auch durch Ozonolyse von α-Methylencarbonsäureestern der allgemeinen Formel IV

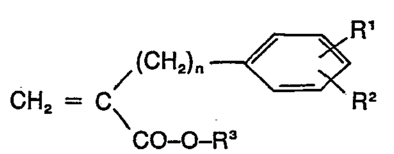
(IV),

worin $R^1$, $R^2$, $R^3$ und n die oben angegebene Bedeutung hat, erfolgen. Die α-Methylencarbonsäureester IV (bzw. die entsprechenden Ausgestaltungen IV* und IV**) werden in Analogie zu den von H. Stetter und H. Kuhlmann [Synthesis 1978, 29], Ph.E. Pfeffer et al. [J. Org. Chem. 37 (1972) 1256] und W.S. Wadsworth, jun. und W.D. Emmons [J. Amer. Chem. Soc. 83 (1961) 1733] beschriebenen Methoden hergestellt.

Die Herstellung der Diester III erfolgt nach Methoden wie sie dem Fachmann geläufig sind, beispielsweise durch Umsetzung von Carbonsäureestern V mit Dialkyloxalat VI

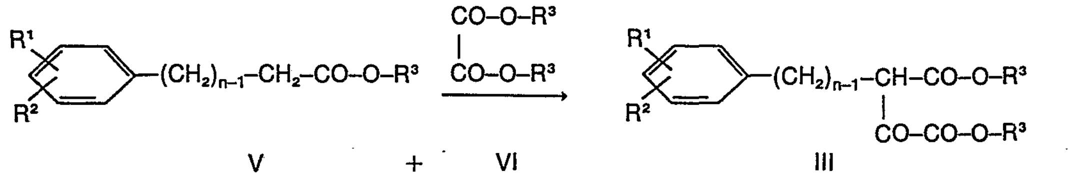

V + VI III

wobei $R^1$, $R^2$, $R^3$ und n die oben angegebene Bedeutung haben, in Gegenwart einer starken Base, z.B. Natriumethanolat oder Kalium-tert.-butylat. Die Carbonsäureester V sind nach bekannten Methoden, z.B. nach R. Huisgen et al. Liebigs Annalen 586(1974)52, zugänglich.

Die folgenden Beispiele dienen zur Erläuterung der Erfindung, ohne sie zu beschränken. Kp. bedeutet Siedepunkt, F. bedeutet Schmelzpunkt. Temperaturangaben erfolgen in °C.

## Beispiele

### Beispiel 1

2-Oxo-6-phenylcapronsäure

a) 10,0 g des nach b) erhaltenen Diesters werden zusammen mit 100 ml 6 N Salzsäure und 100 ml Dioxan 4 Stunden auf ca. 100 °C erhitzt. Nach Abkühlen verdünnt man mit 1,2 l Wasser und extrahiert mehrmals mit Methylenchlorid. Die organische Phase wird mit Wasser und gesättigter Kochsalzlösung gewaschen, dann getrocknet und eingeengt. Der ölige Rückstand wird durch Hochvakuumdestillation [Kp. 130–135 °C bei 13,3 Pa (0,1 Torr)] und Säulenchromatographie (Laufmittel: Chloroform) gereinigt. Es verbleiben 2,9 g 2-Oxo-6-phenylcapronsäure als nicht kristallisierendes Öl, Ausbeute 43% (d.Th.).

b) Zu einer Natriumethylatsuspension, hergestellt aus 6,4 g Natrium und 100 ml Ethanol mit anschliessendem Abdampfen überschüssigen Ethanols und Zugabe von 100 ml Toluol, gibt man 47,9 g 5-Phenylvaleriansäureethylester. Unter Rühren tropft man 44,1 g Diethyloxalat zu und erhitzt 2,5 Stunden auf Rückfluss. Nach Abkühlen wird die Lösung mit 500 ml Eiswasser verrührt und mit 2 N Schwefelsäure auf pH 2 angesäuert. Mehrfache Extraktion mit Methylenchlorid ergibt nach Trocknen, Filtration durch eine kurze Kieselgel-Säule und Einengen 62,1 g 3-Ethoxycarbonyl-2-oxo-6-phenylhexansäureethylester, der ohne weitere Reinigung gemäss a) umgesetzt wird.

### Beispiel 2

6-(4-Methoxyphenyl)-2-oxocapronsäure

a) Analog Beispiel 1a) hydrolysiert und decarboxyliert man den nach b) erhaltenen Diester mit 6 N Salzsäure. Die gesammelten Methylenchloridextrakte engt man ein, löst den Rückstand in Diethylether und extrahiert mehrmals mit 1 N Natronlauge. Die wässrigen Phasen werden mit 6 N Salzsäure auf pH 5,5 angesäuert und dreimal mit Diethylether gewaschen. Darauf bringt man die wässrige Phase auf pH 1–2 und extrahiert mit Methylenchlorid. Nach Trocknen und Einengen verbleibt ein zähes Öl, das aus Petrolether (50–70 °C) in der Kälte kristallisiert. Ausbeute 6,2 g (51% d.Th.), F. 22–25 °C.

b) Analog Beispiel 1b) werden 17,2 g 3-Ethoxycarbonyl-6-(4-methoxyphenyl)-2-oxocapronsäureethylester aus 14,7 g 5-(4-Methoxyphenyl)-valeriansäureethylester und 11,8 g Diethyloxalat mit Natriumethylat als Base hergestellt.

### Beispiel 3

5-(4-Chlorphenyl)-2-oxovaleriansäure

a) 5-(4-Chlorphenyl)-2-oxovaleriansäureethylester (Rückstand aus b) wird in 100 ml 6 N Salzsäure und 100 ml Dioxan aufgenommen und 3 Stunden auf 100° erhitzt. Nach Abkühlen verdünnt man mit 1 l Wasser und extrahiert mehrmals mit Methylenchlorid. Die organische Phase wird mehrmals mit Wasser gewaschen, über Magnesiumsulfat getrocknet und eingeengt. Der Rückstand wird im Kugelrohrofen hochvakuumdestilliert und aus Petrolether umkristallisiert. Man erhält 5,7 g der Titelverbindung vom F. 82–84 °C.

b) 14 g 5-(4-Chlorphenyl)-2-methylenvaleriansäureethylester werden in 350 ml Ethanol gelöst und mit der äquivalenten Menge Ozon behandelt. Nach Spülen der Apparatur mit Stickstoff gibt man ca. 1 g Palladium auf Kohle (10% Pd) zu und reduziert das entstandene Ozonid im Umlaufverfahren mit Wasserstoff. Anschliessend filtriert man den Katalysator ab und engt die Lösung im Vakuum ein.

c) Analog der von H. Stetter und H. Kuhlmann [Synthesis 1979, 29] beschriebenen Methode erhält man aus 71 g 3-(4-Chlorphenyl)-propylmalonsäuremonoethylester, 10,42 g Paraformaldehyd, 47 ml Pyridin und 3,1 ml Piperidin 53,3 g 5-(4-Chlorphenyl)-2-methylenvaleriansäureethylester vom Kp. 120–123 °C bei 6,65 Pa (0,05 Torr).

d) Man tropft bei Raumtemperatur eine Lösung von 16,8 g Kaliumhydroxid in 400 ml Ethanol zu 92 g 3-(4-Chlorphenyl)-propylmalonsäurediethylester in 200 ml Ethanol. Man rührt 24 Stunden, engt im Vakuum weitgehend ein, nimmt den Rückstand in 500 ml Wasser auf und extrahiert 2 mal mit je 100 ml Diethylether. Die wässerige Phase wird unter Eiskühlung mit konzentrierter Salzsäure angesäuert und 3mal mit je 200 ml Diethylether extrahiert; die organische Phase wird nach dem Trocknen über Natriumsulfat eingeengt. Zurück bleiben 71,8 g 3-(4-Chlorphenyl)-propylmalonsäureethylester als viskoses Öl.

e) Man tropft bei 50 °C 108,5 g Malonsäurediethylester zu einer aus 15,6 g Natrium und 750 ml Ethanol frisch hergestellten Natriumethylatlösung. Man hält 2,5 Stunden bei dieser Temperatur

und tropft anschliessend 220 g p-Toluolsulfonsäure-[3-(4-chlorphenyl)-propyl]-ester zu. Nach beendeter Zugabe rührt man 6 Stunden bei 50 °C, versetzt anschliessend mit 800 ml Wasser und extrahiert 3 mal mit insgesamt 1 Liter Diethylether. Die vereinigten organischen Phasen werden nach dem Trocknen über Natriumsulfat und Abdampfen des Lösungsmittels destilliert. Man erhält 105,6 g 3-(4-Chlorphenyl)-propylmalonsäurediethylester vom Kp. 145–155 °C bei 1,33 Pa (0,01 Torr).

f) Man tropft bei 0 °C 135 ml Pyridin zu 150 g 3-(4-Chlorphenyl)-propanol-1 und 206,6 g p-Toluolsulfonsäurechlorid in 300 ml Chloroform. Nach beendeter Zugabe rührt man 3 Stunden bei Raumtemperatur und giesst die Lösung in eine Mischung aus 400 ml Wasser und 120 ml konzentrierter Salzsäure. Die organische Phase wird abgetrennt, 3 mal mit Wasser gewaschen, über Natriumsulfat getrocknet und im Vakuum zu einem gelblichen viskosen Öl eingeengt, Ausbeute 285 g p-Toluolsulfonsäure-[3-(4-chlorphenyl)-propyl]-ester.

Beispiel 4

2-Oxo-7-(3-trifluormethylphenyl)-heptansäure

a) Analog Beispiel 1a) hydrolysiert und decarboxyliert man den nach b) erhaltenen Diester mit 6 N Salzsäure in Dioxan bei 100 °C. Nach extraktiver Aufarbeitung und Reinigung durch Kugelrohrdestillation erhält man 5,6 g der Titelverbindung als zähes, farbloses Öl, das nicht kristallisiert.

b) Analog Beispiel 1b) wird 3-Ethoxycarbonyl-2-oxo-7-(3-trifluormethylphenyl)-heptansäureethylester aus 10,9 g 6-(3-Trifluormethylphenyl)-capronsäureethylester und 6 g Diethyloxalat mit Natriumethylat als Base erhalten, Ausbeute 11,7 g (80% d.Th.).

c) 11,4 g 6-(3-Trifluormethylphenyl)-capronsäure werden in 200 ml Ethanol gelöst und nach Zugabe von 2 ml konzentrierter Schwefelsäure 6 Stunden auf Rückfluss erhitzt. Nach Abdestillation der Hauptmenge des Lösungsmittels wird mit Wasser versetzt und mit Methylenchlorid extrahiert. Behandeln mit Natriumhydrogencarbonatlösung, Trocknen und Einengen ergibt 12,6 g des entsprechenden Ethylesters als Öl, der ohne weitere Reinigung in b) eingesetzt wird.

d) 24,9 g 4-(3-Trifluormethylphenyl)-butylmalonsäurediethylester werden mit 4,5 g Kaliumhydroxid in 200 ml Toluol : Methanol (2 : 1) 36 Stunden am Rückfluss erhitzt und anschliessend sauer extrahiert. Der nach Trocknen und Einengen verbleibende Rückstand wird im Vakuum 2 Stunden auf ca. 170 °C erhitzt; dabei wird Kohlendioxyd frei und es bildet sich die entsprechende Capronsäure, die in c) eingesetzt wird.

e) In eine aus 28,5 g 2-(3-Trifluormethylphenyl)-ethylbromid und 2,9 g Magnesium hergestellte Grignard-Lösung in 200 ml Diethylether tropft man 5,5 g Oxiran in 20 ml Diethylether bei ca. 0 °C. Nach 1 Stunde Rühren und Versetzen mit 100 ml 10%iger Schwefelsäure extrahiert man mit Diethylether und destilliert den Rückstand. Man erhält ca. 18,5 g 4-(3-Trifluormethylphenyl)-butan-1-ol, das in 70 ml Toluol gelöst und mit 16 g p-Toluolsulfonsäurechlorid und 25 ml Pyridin versetzt und gerührt wird. Nach 2 Tagen wird vom Niederschlag abfiltriert und extraktiv aufgearbeitet. Nach Einengen der organischen Phase bleiben 25,7 g Tosylat des substituierten Butanols zurück, das in 250 ml Ethanol aufgenommen und zu einer aus 11,5 g Diethylmalonat und 1,6 g Natrium in 220 ml absolutem Ethanol hergestellten Lösung zugetropft wird. Die Mischung wird 24 Stunden auf Rückfluss erhitzt, danach eingeengt und zwischen Wasser und Methylenchlorid verteilt. Nach mehrmaliger Extraktion werden die gesammelten organischen Phasen getrocknet und eingeengt. Der Rückstand wird über eine 500 g Kieselgelsäule chromatografisch gereinigt und ergibt 24,9 g 4-(3-Trifluormethylphenyl)-butylmalonsäurediethylester, der in d) eingesetzt wird.

Beispiel 5

9-(2,4-Dimethylphenyl)-2-oxo-nonansäure

a) 8,6 g des nach b) erhaltenen Diesters werden nach der in Beispiel 1a) genannten Methode mit 6 N Salzsäure und Dioxan hydrolysiert und decarboxyliert. Extraktion mit Methylenchlorid, gefolgt von Waschen, Trocknen und Einengen, liefert nach Reinigung durch Hochvakuumdestillation 4,9 g der Titelverbindung als zähes Öl.

b) 7,5 g des nach c) erhaltenen Octansäureesters werden analog Beispiel 1b) mit Natriumethylat und Diethyloxalat umgesetzt. Nach Ansäuern, Extrahieren mit Methylenchlorid, Trocknen und Filtrieren durch Kieselgel verbleiben nach Entfernen des Lösungsmittels 8,6 g 9-(2,4-Dimethylphenyl)-3-ethoxycarbonyl-2-oxononansäureethylester.

c) 12,8 g 8-(2,4-Dimethylphenyl)-7-oxooctansäureethylester [erhalten nach d)] werden analog der Methode von Huisgen [R. Huisgen et al., Liebigs Ann. 586 (1954)52] mit Hydrazinhydrat in Diethylenglykol reduziert. Das isolierte Reduktionsprodukt wird in 200 ml Ethanol und 5 ml konzentrierter Schwefelsäure über Nacht zum Rückfluss erhitzt und liefert nach üblicher Aufarbeitung 7,5 g 8-(2,4-Dimethylphenyl)-octansäureethylester als farbloses Öl.

d) Aus 14,5 g 2,4-Dimethylbenzylbromid wird nach der Methode von Huisgen mit Magnesium und Cadmiumchlorid das entsprechende Cadmium-dialkyl hergestellt. Anschliessend setzt man dieses in benzolischer Lösung mit 11,2 g Pimelinsäureethylesterchlorid in der Siedehitze um. Nach Versetzen mit Schwefelsäure wird die organische Phase gewaschen und das Benzol abgedampft. Der Rückstand wird im Hochvakuum destilliert und liefert 12,8 g 8-(2,4-Dimethylphenyl)-7-oxooctansäureethylester.

Beispiel 6

10-(4-Chlorphenyl)-2-oxodecansäure

a) 5,7 g des nach b) erhaltenen Diesters werden nach Beispiel 1a) mit 6 N Salzsäure in Dioxan hydrolysiert und decarboxyliert. Nach Aufarbeitung und Reinigung durch Säulenchromatographie verbleiben 3,8 g der Titelverbindung als farbloses Öl.

b) 5,3 g der nach c) erhaltenen Verbindung werden nach Beispiel 1b) mit Diethyloxalat und Natriumethylat umgesetzt. Aufarbeitung und Reinigung durch Kieselgel-Filtration liefern nach Eindampfen des Lösungsmittels 5,7 g 10-(2,4-Dimethylphenyl)-3-ethoxycarbonyl-2-oxodecansäureethylester.

c) 8,3 g 9-(4-Chlorphenyl)-9-oxo-nonansäureethylester [erhalten nach d)] werden analog Beispiel 5c) mit Hydrazinhydrat reduziert und aufgearbeitet. Man erhält 5,3 g 9-(4-Chlorphenyl)-nonansäureethylester als Öl.

d) Nach der von Papa et al. [J. Amer. Chem. Soc. 69 (1947) 3018] beschriebenen Methode werden 12,3 g Azelainsäureethylesterchlorid in 20 ml Chlorbenzol mit 9,5 g Aluminiumtrichlorid in der Kälte zusammengegeben und dann über Nacht auf 150 °C erhitzt. Nach Versetzen des Komplexes mit verdünnter Salzsäure wird die organische Phase gewaschen und eingeengt; der Rückstand wird mit Diethylether extrahiert. Nach Säulenchromatographie und Einengen der Lösung werden 8,3 g 9-(4-Chlorphenyl)-9-oxo-nonansäureethylester als zähes Öl erhalten.

Beispiel 7

6-(4-Methoxyphenyl)-2-oxocapronsäure-Natriumsalz

4,5 g 6-(4-Methoxyphenyl)-2-oxocapronsäure werden in 19,0 ml 1 N Natronlauge 15 Minuten bei Raumtemperatur aufgerührt. Die Mischung wird filtriert, einmal mit Diethylether gewaschen und zur Trockne eingedampft. Der bei 30 °C im Vakuum getrocknete Rückstand besteht aus dem reinen Natriumsalz.

Beispiel 8

5-(4-Chlorphenyl)-2-oxovaleriansäure-calciumsalz

5,3 g 5-(4-Chlorphenyl)-2-oxovaleriansäure werden in 25 ml 1 N Natronlauge gelöst und mit wenig halbkonzentrierter Salzsäure auf pH 8,5 eingestellt. Durch Zugabe von 2,0 g Calciumchloriddihydrat in 6 ml Wasser unter Rühren wird das Calciumsalz gefällt, das nach Filtrieren im Vakuum bei 40 °C getrocknet wird.

Beispiel 9

10 000 Kapseln mit einem Wirkstoffgehalt von 25 mg werden wie folgt hergestellt:

250 g 5-(4-Chlorphenyl)-2-oxovaleriansäure werden in 3000 ml Methylenchlorid gelöst. Die Lösung wird mit 750 g mikronisierter Kieselsäure gut gemischt. Die Mischung wird zur Trockne eingedampft und dann in Hartgelatinekapseln Grösse 4 abgefüllt.

Beispiel 10

10 000 Tabletten mit einem Wirkstoffgehalt von 30 mg werden wie folgt hergestellt:

300 g 5-(4-Chlorphenyl)-2-oxovaleriansäure, 800 g Xylit, 500 g Calciumphosphat, 30 mg amorphe Kieselsäure und 40 g Natriumlaurylsulfat werden gemischt und gesiebt. Diese Mischung wird mit einer Lösung von 50 g Polyvinylpyrrolidon (mittleres Mol.-Gewicht 25 000) in 320 ml Ethanol befeuchtet und durch ein Sieb mit der Maschenweite 1,25 mm granuliert. Das Granulat wird bei 40 °C getrocknet und mit 160 g Pektin, 100 g Talk und 20 g Magnesiumstearat gemischt. Diese Mischung wird zu Tabletten à 200 mg mit 8 mm Durchmesser verpresst.

Beispiel 11

10 000 Kapseln mit einem Wirkstoffgehalt von 25 mg werden wie folgt hergestellt:

250 g 5-(4-Methoxyphenyl)-2-oxovaleriansäure, 495 g mikrokristalline Cellulose und 255 g amorphe Kieselsäure werden gut gemischt und in Hartgelatinekapseln Grösse 4 abgefüllt.

Pharmakologie

Die erfindungsgemässen Verbindungen senken infolge ihrer insulinotropen Wirkung den Blutglukosespiegel, wobei sie sich in ihrer chemischen Struktur grundlegend von pankreaswirksamen, betacytotropen Substanzen (z.B. Sulfonylharnstoffen) unterscheiden. Als besonders vorteilhaft erweist sich, dass der insulinotrope Effekt der Verbindungen im Gegensatz zu handelsüblichen Sulfonylharnstoffen von der Glukosekonzentration des umgebenden Mediums abhängig ist. Die erfindungsgemässen Verbindungen zeigen somit die Voraussetzungen für ein sogenanntes «denkendes Antidiabetikum», das die Insulinabgabe nur bei Hyperglykämie stimuliert, während es bei euglykämischen Bedingungen keine zusätzliche Insulinabgabe hervorruft. Unter solchen Voraussetzungen wird die Gefahr einer Hypoglykämie ausgeschlossen.

In der anschliessenden Tabelle werden die untersuchten Verbindungen durch eine laufende Nummer bezeichnet, die wie folgt zuzuordnen ist:

| Lfd. Nr. | Name der Verbindung |
|---|---|
| 1 | Tolbutamid [$N^1$-n-Butyl-$N^2$-(4-methylphenylsulfonyl)-harnstoff] |
| 2 | 5-(4-Methoxyphenyl)-2-oxo-valeriansäure |
| 3 | 6-(4-Methoxyphenyl)-2-oxo-capronsäure |
| 4 | 5-(4-Chlorphenyl)-2-oxovaleriansäure |

In der Tabelle I werden Untersuchungen der Insulinsekretion aus dem isoliert perfundierten Rattenpankreas innerhalb 60 Minuten bei alleiniger Zugabe von Glukose und bei Zusatz von Vertretern der erfindungsgemässen Verbindungen zum Perfusat wiedergegeben.

Tabelle 1
Insulinausschüttung aus dem isoliert perfundierten Rattenpankreas

| Zusätze zum Perfusat | | Insulinabgabe I | Verstärkungseffekt |
|---|---|---|---|
| Verb.-Nr. (in [mmol/Liter]) | Glukose [mmol/Liter] | [ng/60 Min.] | $I^+/I^-$ |
| – | 4 | 23 | – |
| – | 8,3 | 55 | – |
| 1(1) | 4 | 240 | 10,4 |
| 1(1) | 8,3 | 470 | 8,5 |
| 2(0,42) | 4 | 23 | 1 |
| 2(0,42) | 8,3 | 260 | 4,7 |
| 3(0,42) | 8,3 | 270 | 4,9 |
| 4(0,42) | 8,3 | 430 | 7,8 |

Zu Tabelle I:
$I^+$ = Insulinabgabe bei Zusatz der Testverbindungen und Glukose
$I^-$ = Insulinabgabe ohne Zusatz der Testverbindungen, jedoch bei Zusatz von Glukose

Aus Tabelle I ist ersichtlich, dass die Insulinabgabe bei alleinigem Zusatz von Glukose bei einer Konzentration von 8,3 mmol/l gegenüber dem von 4 mmol/l um den Faktor 2,3 gesteigert ist. Gleichzeitiger Tolbutamid-Zusatz steigert die Insulinausschüttung bei diesen Glukosekonzentrationen um die Faktoren 10,4 bzw. 8,5. Die erfindungsgemässe Verbindung 2 dagegen ist bei einer Glukosekonzentration von 4 mmol/l ohne Einfluss auf die Insulinsekretion, während sie bei einer Glukosekonzentration von 8,3 mmol/l eine deutliche Steigerung der Insulinausschüttung (auf das 4,7 fache) bewirkt. Eine fast gleich starke bzw. eine höhere Steigerungsrate der Insulinabgabe werden bei Zusatz der erfindungsgemässen Verbindungen 3 bzw. 4 erzielt.

Die Ermittlung der pharmakologischen Eigenschaften erfolgte nach folgender Methode:

A. Versuchstiere
Als Versuchstiere dienen gefastete, männliche Sprague-Dawley-Ratten (220–280 g) vom Stamm mus rattus, die unter Standardbedingungen (Tag-Nachtrhythmus 12h/12h, 23 °C, 55% Luftfeuchtigkeit, Wasser und Standarddiät Altromin[R] ad libitum) gehalten werden. 18 bis 20 Stunden vor Versuchsbeginn wird den Ratten das Futter entzogen.

B. Perfusionsmedium
Die Perfusion des Rattenpankreas wird analog der von Lenzen beschriebenen Methode [Amer. J. Physiol. 236(1979)E391–E400] durchgeführt. Als Perfusionsmedium wird Krebs-Henseleit-Puffer verwendet, der 1 mg/ml Rinderserumalbumin (Serva) und Glukose, wie angegeben, enthält. Das Perfusionsmedium wird in Gaswaschflaschen mit Carbogen (95%$O_2$/5%$CO_2$) ständig bei 37,5 °C und pH 7,4 equilibriert.

C. Perfusion und Operation
Die Ratten werden mit Nembutal (0,8 ml/kg) anästhesiert. Das Pankreas wird mit dem Magen, der anliegenden Duodenalschleife sowie dem versorgenden und ableitenden Gefässsystem chirurgisch isoliert. Die Kanülierung erfolgt retrograd durch die Aorta. Alle abzweigenden Gefässe werden so ligiert, dass die Perfusionslösung durch die arteria coeliaca den Pankreas erreicht, ihn durchströmt, in der vena lienalis und der vena pancreatico-duodenalis gesammelt wird und dann in die vena porta mündet. Die Poartalvene wird wiederum retrograd kanüliert und hier wird das Perfusat fraktioniert aufgefangen. Das isolierte Organpräparat wird in ein thermostatisiertes Glasgefäss mit einer am Boden gelegenen Öffnung zur Aufnahme der Ablaufkapillare überführt. Das Pankreas wird mit einem konstanten Fluss von 4 ml/min. infundiert. Wenn mindestens 3,7 ml wieder aufgefangen werden, wird das Präparat zum Experiment verwendet. Nach einer Vorperfusion von 10 Minuten, in der das Pankreas blutfrei gespült wird, wird für die Dauer von 20 Minuten ohne Substanz bei angegebener Glucosekonzentration und für die Dauer von 60 Minuten mit konstanter Testsubstanzkonzentration und der angegebenen Glucosekonzentration perfundiert. Das Perfusat wird jeweils zunächst für die Dauer von 10 Minuten in 2 Minuten-Abständen und darauffolgend in 5 Minuten-Abständen gesammelt.

D. Insulinbestimmung
Die Insulinbestimmung wird mit dem Radioimmunoassay der Firma Becton und Dickinson durchgeführt. Dieser Test arbeitet mit Meerschweinchenantikörpern gegen Schweineinsulin und mit $^{125}$I-markiertem Schweineinsulin. Das freie Antigen wird in diesem Test durch Adsorption an Dextran-beschichtete Aktivkohle und nachfolgende Zentrifugation getrennt. Als Standard für die Eichkurve wird hochreines Ratteninsulin der Firma Novo verwendet.

Aus den Insulinkonzentrationen der einzelnen Perfusatfraktionen wird die innerhalb 60 Minuten ausgeschiedene Menge an Insulin errechnet. Bei Perfusion ohne Substanz erwiesen Vorversuche, dass die Insulinsekretion nach 10 Minuten konstant bleibt. Der Efflux zwischen Minute 11 bis 60 wird dann aus dem Efflux zwischen Minute 11 bis 20 extrapoliert. Das Präparat wird dadurch kürzere Zeit belastet, gleichzeitig erhält man für jeden Versuch die Kontrolle im gleichen Experiment.

**Patentansprüche für die Vertragsstaaten: BE, CH, DE, FR, GB, IT, LI, LU, NL, SE**

1. Substituierte Oxocarbonsäuren der allgemeinen Formel I

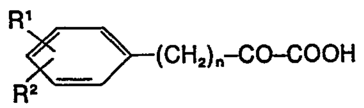
(I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat,

und ihre Salze.

2. Verbindungen nach Anspruch 1, worin n eine ganze Zahl von 3 bis 6 bedeutet, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat.

3. Substituierte Oxocarbonsäuren nach Anspruch 1, gekennzeichnet durch die allgemeine Formel I*

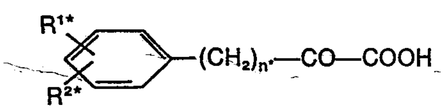
(I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom, eine Methylgruppe oder eine Methoxygruppe und $R^{2*}$ ein Wasserstoffatom bedeuten, wenn n* die Bedeutung 3 hat,

und ihre Salze.

4. Substituierte Oxocarbonsäuren nach Anspruch 1, gekennzeichnet durch die allgemeine Formel I**

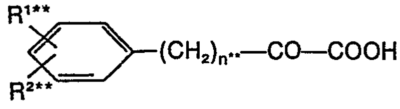
(I**),

worin

$R^{1**}$ und $R^{2**}$ in meta- oder para-Stellung zum Ketocarbonsäurerest stehen und

$R^{1**}$ ein Wasserstoffatom oder ein Chloratom,

$R^{2**}$ ein Chloratom und

n** 3 bis 5 bedeuten,

und ihre pharmakologisch verträglichen Salze mit anorganischen oder organischen Basen.

5. Substituierte Oxocarbonsäuren der allgemeinen Formel I′

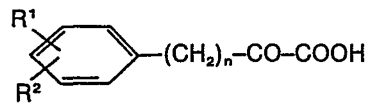
(I′),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und ihre pharmakologisch verträglichen Salze mit anorganischen oder organischen Basen zur Anwendung bei der Behandlung von Krankheiten, die auf Störungen des Glucosestoffwechsels beruhen.

6. 5-(4-Methoxyphenyl)-2-oxovaleriansäure und ihre pharmakologisch verträglichen Salze mit anorganischen oder organischen Basen zur Verwendung bei der Behandlung von Krankheiten, die auf Störungen des Glucosestoffwechsels beruhen.

7. Arzneimittel, die eine oder mehrere der substituierten Oxocarbonsäuren der allgemeinen Formel I′

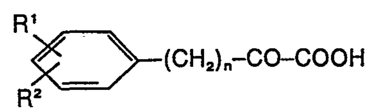
(I′),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und/oder ihre pharmakologisch verträglichen Salze mit anorganischen oder organischen Basen enthalten.

8. Arzneimittel nach Anspruch 7, enthaltend substituierte Oxocarbonsäuren der allgemeinen Formel I′, in der $R^1$ ein Wasserstoffatom, ein Chloratom, eine Methylgruppe, eine Methoxygruppe oder eine Trifluormethylgruppe, $R^2$ ein Wasserstoffatom oder ein Chloratom und n eine ganze Zahl von 3 bis 6 bedeuten, und/oder ihre pharmakologisch verträglichen Salze.

9. Arzneimittel nach Anspruch 7, enthaltend Verbindungen nach Anspruch 3.

10. Arzneimittel nach Anspruch 7, enthaltend Verbindungen nach Anspruch 4.

11. Verfahren zur Herstellung von substituierten Oxocarbonsäuren der allgemeinen Formel I

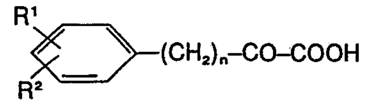 (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat,

und ihren Salzen, dadurch gekennzeichnet, dass man

a) einen Oxocarbonsäureester der allgemeinen Formel II

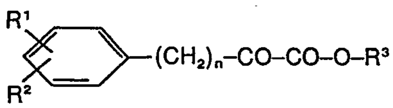 (II),

worin $R^1$, $R^2$ und n die vorstehend angegebene Bedeutung haben und $R^3$ einen Niederalkylrest bedeutet, solvolysiert und gegebenenfalls anschliessend erhaltene Säuren in die Salze oder erhaltene Salze in die Säuren überführt, oder

b) einen Diester der allgemeinen Formel III

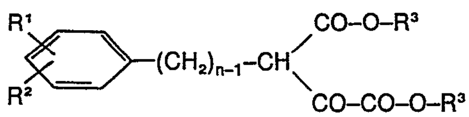 (III),

worin $R^1$, $R^2$, $R^3$ und n die vorstehend angegebene Bedeutung haben, solvolysiert und decarboxyliert und gegebenenfalls anschliessend erhaltene Säuren in die Salze oder erhaltene Salze in die Säuren überführt.

12. Verfahren nach Anspruch 11, dadurch gekennzeichnet, dass man Oxocarbonsäureester II oder Diester III als Ausgangsverbindungen einsetzt, in denen n eine ganze Zahl von 3 bis 6 bedeutet, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat.

13. Verfahren nach Anspruch 11 zur Herstellung von substituierten Oxocarbonsäuren der allgemeinen Formel I*

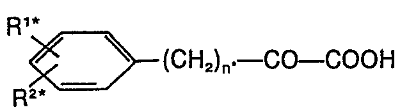 (I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom, eine Methylgruppe oder eine Methoxygruppe und $R^{2*}$ ein Wasserstoffatom bedeuten, wenn n* die Bedeutung 3 hat,

und ihren Salzen, dadurch gekennzeichnet, dass man

a) einen Oxocarbonsäureester der allgemeinen Formel II*

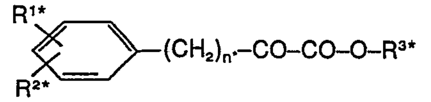 (II*)

worin $R^{1*}$, $R^{2*}$ und n* die oben angegebene Bedeutung haben und $R^{3*}$ einen Niederalkylrest bedeutet, solvolysiert und gegebenenfalls anschliessend erhaltene Säuren in die Salze oder erhaltene Salze in die Säuren überführt, oder

b) einen Diester der allgemeinen Formel III*

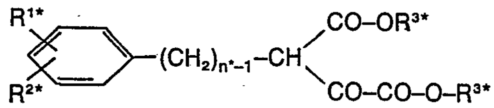 (III*)

worin $R^{1*}$, $R^{2*}$ und n* die oben angegebene Bedeutung haben und $R^{3*}$ einen Niederalkylrest bedeutet, solvolysiert und decarboxyliert und gegebenenfalls anschliessend erhaltene Säuren in die Salze oder erhaltene Salze in die Säuren überführt.

14. Verfahren nach Anspruch 11 zur Herstellung von substituierten Oxocarbonsäuren der allgemeinen Formel I**

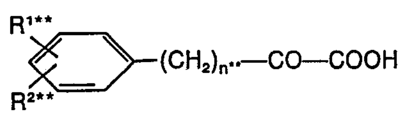 (I**),

worin $R^{1**}$ und $R^{2**}$ in meta- oder para-Stellung zum Ketocarbonsäurerest stehen und

$R^{1**}$ ein Wasserstoffatom oder ein Chloratom,

$R^{2**}$ ein Chloratom und

n** 3 bis 5 bedeuten,

und ihren Salzen, dadurch gekennzeichnet, dass man

a) einen Oxocarbonsäureester der allgemeinen Formel II**

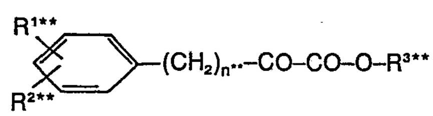 (II**)

worin $R^{1**}$, $R^{2**}$ und n** die oben angegebene Bedeutung haben und $R^{3**}$ einen Methyl- oder Ethylrest bedeutet, solvolysiert und gegebenenfalls anschliessend erhaltene Säuren in die Salze oder erhaltene Salze in die Säuren überführt, oder

b) einen Diester der allgemeinen Formel III**

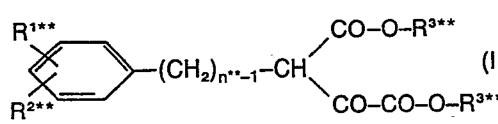 (III**)

worin $R^{1**}$, $R^{2**}$ und n** die oben angegebene Bedeutung haben und $R^{3**}$ einen Methyl- oder Ethylrest bedeutet, solvolysiert und decarboxyliert und gegebenenfalls anschliessend erhaltene Säuren in die Salze oder erhaltene Salze in die Säuren überführt.

**Patentansprüche für den Vertragsstaat AT**

1. Verfahren zur Herstellung von substituierten Oxocarbonsäuren der allgemeinen Formel I

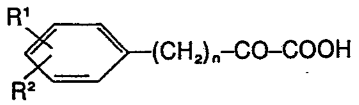 (I),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat,

und ihren Salzen, dadurch gekennzeichnet, dass man

a) einen Oxocarbonsäureester der allgemeinen Formel II

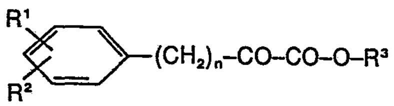 (II),

worin $R^1$, $R^2$ und n die vorstehend angegebene Bedeutung haben und $R^3$ einen Niederalkylrest bedeutet, solvolysiert und gegebenenfalls anschliessend erhaltene Säuren in die Salze oder erhaltene Salze in die Säuren überführt, oder

b) einen Diester der allgemeinen Formel III

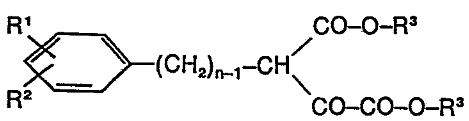 (III),

worin $R^1$, $R^2$, $R^3$ und n die vorstehend angegebene Bedeutung haben, solvolysiert und decarboxyliert und gegebenenfalls anschliessend erhaltene Säuren in die Salze oder erhaltene Salze in die Säuren überführt.

2. Verfahren nach Anspruch 1, dadurch gekennzeichnet, dass man Oxocarbonsäureester II oder Diester III als Ausgangsverbindungen einsetzt, in denen n eine ganze Zahl von 3 bis 6 bedeutet, wobei nicht $R^1$ ein Wasserstoffatom, ein Fluoratom, eine Methylgruppe oder eine Methoxygruppe und $R^2$ ein Wasserstoffatom bedeuten, wenn n die Bedeutung 3 hat.

3. Verfahren nach Anspruch 1 zur Herstellung von substituierten Oxocarbonsäuren der allgemeinen Formel I*

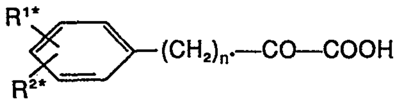 (I*),

worin

$R^{1*}$ ein Wasserstoffatom, ein Chloratom, eine Methylgruppe, eine Methoxygruppe oder eine Trifluormethylgruppe,

$R^{2*}$ ein Wasserstoffatom oder ein Chloratom und

n* eine ganze Zahl von 3 bis 6 bedeuten, wobei nicht $R^{1*}$ ein Wasserstoffatom, eine Methylgruppe oder eine Methoxygruppe und $R^{2*}$ ein Wasserstoffatom bedeuten, wenn n* die Bedeutung 3 hat,

und ihren Salzen, dadurch gekennzeichnet, dass man

a) einen Oxocarbonsäureester der allgemeinen Formel II*

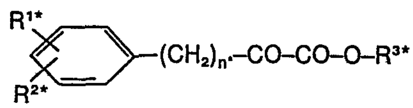 (II*)

worin $R^{1*}$, $R^{2*}$ und n* die oben angegebene Bedeutung haben und $R^{3*}$ einen Niederalkylrest bedeutet, solvolysiert und gegebenenfalls anschliessend erhaltene Säuren in die Salze oder erhaltene Salze in die Säuren überführt, oder

b) einen Diester der allgemeinen Formel III*

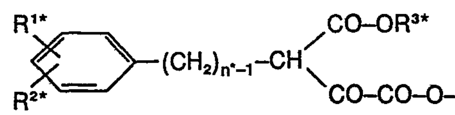 (III*)

worin $R^{1*}$, $R^{2*}$ und n* die oben angegebene Bedeutung haben und $R^{3*}$ einen Niederalkylrest bedeutet, solvolysiert und decarboxyliert und gegebenenfalls anschliessend erhaltene Säuren in die Salze oder erhaltene Salze in die Säuren überführt.

4. Verfahren nach Anspruch 1 zur Herstellung von substituierten Oxocarbonsäuren der allgemeinen Formel I**

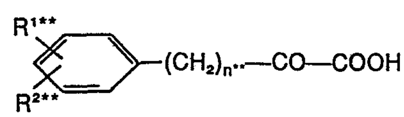 (I**),

worin $R^{1**}$ und $R^{2**}$ in meta- oder para-Stellung zum Ketocarbonsäurerest stehen und

$R^{1**}$ ein Wasserstoffatom oder ein Chloratom,

$R^{2**}$ ein Chloratom und

n** 3 bis 5 bedeuten,

und ihren Salzen, dadurch gekennzeichnet, dass man

a) einen Oxocarbonsäureester der allgemeinen Formel II**

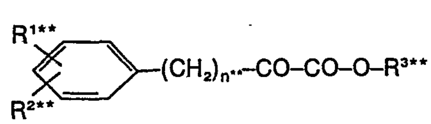 (II**)

worin $R^{1**}$, $R^{2**}$ und n** die oben angegebene Bedeutung haben und $R^{3**}$ einen Methyl- oder Ethylrest bedeutet, solvolysiert und gegebenenfalls anschliessend erhaltene Säuren in die Salze oder erhaltene Salze in die Säuren überführt, oder

b) einen Diester der allgemeinen Formel III**

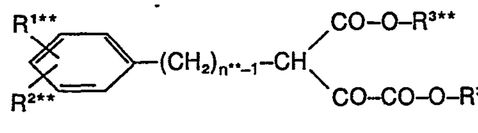 (III**)

worin $R^{1**}$, $R^{2**}$ und $n^{**}$ die oben angegebene Bedeutung haben und $R^{3**}$ einen Methyl- oder Ethylrest bedeutet, solvolysiert und decarboxyliert und gegebenenfalls anschliessend erhaltene Säuren in die Salze oder erhaltene Salze in die Säuren überführt.

5. Verfahren zur Herstellung von neuen Arzneimitteln die als Wirkstoff substituierte Oxocarbonsäuren der allgemeinen Formel I′

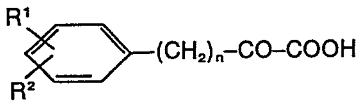

(I′),

worin

$R^1$ ein Wasserstoffatom, ein Halogenatom, eine Hydroxygruppe, eine Niederalkylgruppe, eine Niederalkoxygruppe oder eine Trifluormethylgruppe,

$R^2$ ein Wasserstoffatom oder ein Halogenatom und

n eine ganze Zahl von 3 bis 8 bedeuten,

und/oder ihre pharmakologisch verträglichen Salze mit anorganischen oder organischen Basen enthalten, dadurch gekennzeichnet, dass der Wirkstoff mit einem pharmazeutischen Trägerstoff und gegebenenfalls weiteren Zusatzstoffen gemischt wird.

6. Verfahren nach Anspruch 5, dadurch gekennzeichnet, dass das Arzneimittel in Form einer Einheitsdosis hergestellt wird, die 2 bis 200 mg Wirkstoff enthält.

7. Verfahren nach Anspruch 5 oder 6, dadurch gekennzeichnet, dass der Wirkstoff mit Hilfe eines Verfahrens nach einem der Ansprüche 1 bis 4 hergestellt wurde.

**Claims for the Contracting states BE, CH, DE, FR, GB, IT, LI, LU, NL, SE**

1. Substituted oxocarboxylic acids of the general formula I

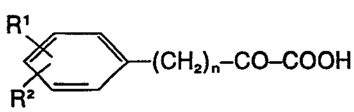

(I)

wherein

$R^1$ denotes a hydrogen atom, a halogen atom, a hydroxyl group, a lower alkyl grup, a lower alkoxy group or a trifluoromethyl group,

$R^2$ denotes a hydrogen atom or a halogen atom and

n denotes an integer from 3 to 8,

excluding those wherein

$R^1$ denotes a hydrogen atom, a fluorine atom, a methyl group or a methoxy group and

$R^2$ denotes a hydrogen atom, if

n denotes 3,

and their salts.

2. Compounds according to Claim 1, wherein n is an integer from 3 to 6, excluding those wherein $R^1$ denotes a hydrogen atom, a fluorine atom, a methyl group or a methoxy group and $R^2$ denotes a hydrogen atom, if n denotes 3.

3. Substituted oxocarboxylic acids according to Claim 1, characterised by the general formula I*

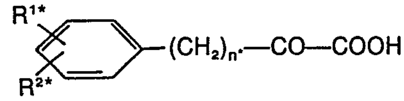

(I*)

wherein

$R^{1*}$ denotes a hydrogen atom, a chlorine atom, a methyl group, a methoxy group or a trifluoromethyl group,

$R^{2*}$ denotes a hydrogen atom or a chlorine atom and

n* denotes an integer form 3 to 6,

excluding those wherein

$R^{1*}$ denotes a hydrogen atom, a methyl group or a methoxy group and

$R^{2*}$ denotes a hydrogen atom, if

n* denotes 3,

and their salts.

4. Substituted oxocarboxylic acids according to Claim 1, characterised by the general formula I**

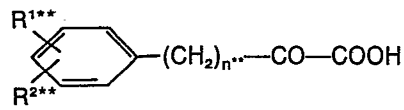

(I**)

wherein

$R^{1**}$ and $R^{2**}$ are in the meta-position or para-position to the ketocarboxylic acid radical, and

$R^{1**}$ denotes a hydrogen atom or a chlorine atom,

$R^{2**}$ denotes a chlorine atom and

n** denotes 3 to 5,

and their pharmacologically acceptable salts with inorganic or organic bases.

5. Substituted oxocarboxylic acids of the general formula I′

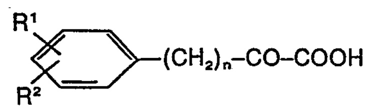

(I′)

wherein

$R^1$ denotes a hydrogen atom, a halogen atom, a hydroxyl group, a lower alkyl group, a lower alkoxy group or a trifluoromethyl group,

$R^2$ denotes a hydrogen atom or a halogen atom and

n denotes an integer from 3 to 8,

and their pharmacologically compatible salts, for use in the treatment of illnesses based on disorders in the glucose metabolism.

6. 5-(4-Methoxyphenyl)-2-oxovaleric acid and their pharmacologically acceptable salts with inorganic or organic bases for use in the treatment of illnesses attributable to disturbances of glucose metabolism.

7. Medicaments which contain one or more of the substituted oxocarboxylic acids of the general formula I′

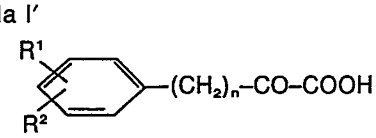

(I′)

wherein

$R^1$ denotes a hydrogen atom, a halogen atom, a hydroxyl group, a lower alkyl group, a lower alkoxy group or a trifluoromethyl group,

$R^2$ denotes a hydrogen atom or a halogen atom and

n denotes an integer from 3 to 8,

and/or their pharmacologically acceptable salts with inorganic or organic bases.

8. Medicaments according to Claim 7, containing substituted oxocarboxylic acids of the general formula I', wherein $R^1$ is a hydrogen atom, a chlorine atom, a methyl group, a methoxy group or a trifluoromethyl group, $R^2$ is a hydrogen atom or a chlorine atom and n is an integer from 3 to 6, and/or their pharmacologically acceptable salts.

9. Medicaments according to Claim 7, containing compounds according to Claim 3.

10. Medicaments according to Claim 7, containing compounds according to Claim 4.

11. Process for the preparation of substituted oxocarboxylic acids of the general formula I

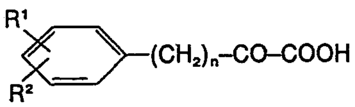 (I)

wherein

$R^1$ denotes a hydrogen atom, a halogen atom, a hydroxyl group, a lower alkyl group, a lower alkoxy group or a trifluoromethyl group,

$R^2$ denotes a hydrogen atom or a halogen atom and

n denotes an integer from 3 to 8,

excluding those wherein

$R^1$ denotes a hydrogen atom, a fluorine atom, a methyl group or a methoxy group and

$R^2$ denotes a hydrogen atom, if

n denotes 3,

and their salts, characterised in that

a) an oxocarboxylic acid ester of the general formula II

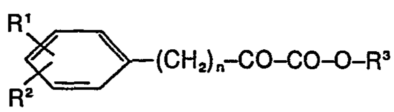 (II)

wherein

$R^1$, $R^2$ and n have the abovementioned meaning and

$R^3$ denotes a lower alkyl radical,

is solvolysed and, if appropriate, resulting acids are subsequently converted into the salts or resulting salts are subsequently converted into the acids, or

b) a diester of the general formula III

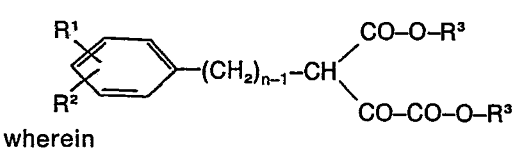 (III)

wherein

$R^1$, $R^2$, $R^3$ and n have the abovementioned meaning, is solvolysed and decarboxylated, and, if appropriate, resulting acids are subsequently converted into the salts or resulting salts are subsequently converted into the acids.

12. Process according to Claim 11, characterised in that oxocarboxylic acid esters II or diesters III, wherein n denotes an integer form 3 to 6, excluding those wherein $R^1$ denotes a hydrogen atom, a fluorine atom, a methyl group or a methoxy group and $R^2$ denotes a hydrogen atom, if n denotes 3, are employed as starting compounds.

13. Process according to Claim 11 for the preparation of substituted oxocarboxylic acids of the general formula I*

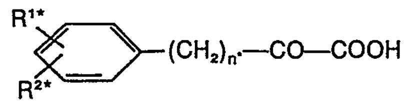 (I*)

wherein

$R^{1*}$ denotes a hydrogen atom, a chlorine atom, a methyl group, a methoxy group or a trifluoromethyl group,

$R^2$ denotes a hydrogen atom or a chlorine atom and

n* denotes an integer from 3 to 6,

excluding those wherein

$R^{1*}$ denotes a hydrogen atom, a methyl group or a methoxy group and

$R^{2*}$ denotes a hydrogen atom, if

n* denotes 3,

and their salts, characterised in that

a) an oxocarboxylic acid ester of the general formula II*

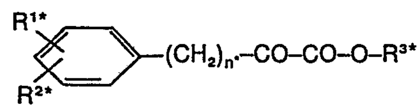 (II*)

wherein

$R^{1*}$, $R^{2*}$ and n* have the abovementioned meaning and

$R^{3*}$ denotes a lower alkyl radical,

is solvolysed and, if appropriate, resulting acids are subsequently converted into the salts or resulting salts are subsequently converted into the acids, or

b) a diester of the general formula III*

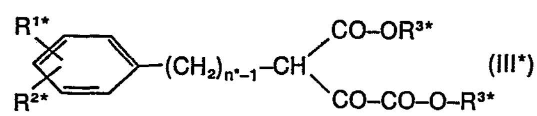 (III*)

wherein

$R^{1*}$, $R^{2*}$ and n* have the abovementioned meaning and

$R^{3*}$ denotes a lower alkyl radical,

is solvolysed and decarboxylated, and, if appropriate, resulting acids are subsequently converted into the salts or resulting salts are subsequently converted into the acids.

14. Process according to Claim 11 for the preparation of substituted oxocarboxylic acids of the general formula I**

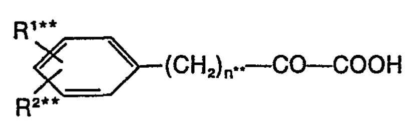 (I**)

wherein

$R^{1**}$ and $R^{2**}$ are in the meta-position or para-position to the ketocarboxylic acid radical, and

$R^{1**}$ denotes a hydrogen atom or a chlorine atom,

$R^{2**}$ denotes a chlorine atom and

$n^{**}$ denotes 3 to 5,

and their salts, characterised in that

a) an oxocarboxylic acid ester of the general formula II**

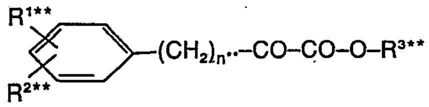

(II**)

wherein

$R^{1**}$, $R^{2**}$ and $n^{**}$ have the abovementioned meaning and

$R^{3**}$ denotes a methyl or ethyl radical,

is solvolysed and, if appropriate, resulting acids are subsequently converted into the salts or resulting salts are subsequently converted into the acids, or

b) a diester of the general formula III**

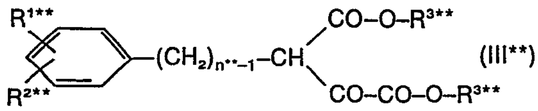

(III**)

wherein

$R^{1**}$, $R^{2**}$ and $n^{**}$ have the abovementioned meaning and

$R^{3**}$ denotes a methyl or ethyl radical,

is solvolysed and decarboxylated, and, if appropriate, resulting acids are subsequently converted into the salts or resulting salts are subsequently converted into the acids.

**Claims for the contracting state AT**

1. Process for the preparation of substituted oxocarboxylic acids of the general formula I

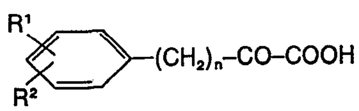

(I)

wherein

$R^1$ denotes a hydrogen atom, a halogen atom, a hydroxyl group, a lower alkyl group, a lower alkoxy group or a trifluoromethyl group,

$R^2$ denotes a hydrogen atom or a halogen atom and

n denotes an integer from 3 to 8,

excluding those wherein

$R^1$ denotes a hydrogen atom, a fluorine atom, a methyl group or a methoxy group and

$R^2$ denotes a hydrogen atom, if

n denotes 3,

and their salts, characterised in that

a) an oxocarboxylic acid ester of the general formula II

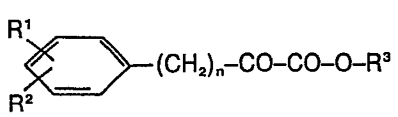

(II)

wherein

$R^1$, $R^2$ and n have the abovementioned meaning and

$R^3$ denotes a lower alkyl radical,

is solvolysed and, if appropriate, resulting acids are subsequently converted into the salts or resulting salts are subsequently converted into the acids, or

b) a diester of the general formula III

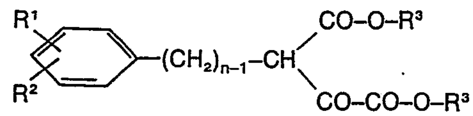

(III)

wherein

$R^1$, $R^2$, $R^3$ and n have the abovementioned meaning, is solvolysed and decarboxylated, and, if appropriate, resulting acids are subsequently converted into the salts or resulting salts are subsequently converted into the acids.

2. Process according to Claim 1, characterised in that oxocarboxylic acid esters II or diesters III, wherein n denotes an integer from 3 to 6, excluding those wherein $R^1$ denotes a hydrogen atom, a fluorine atom, a methyl group or a methoxy group and $R^2$ denotes a hydrogen atom, if n denotes 3, are employed as starting compounds.

3. Process according to Claim 1 for the preparation of substituted oxocarboxylic acids of the general formula I*

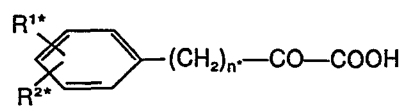

(I*)

wherein

$R^{1*}$ denotes a hydrogen atom, a chlorine atom, a methyl group, a methoxy group or a trifluoromethyl group,

$R^{2*}$ denotes a hydrogen atom or a chlorine atom and

$n^*$ denotes an integer from 3 to 6,

excluding those wherein

$R^{1*}$ denotes a hydrogen atom, a methyl group or a methoxy group and

$R^{2*}$ denotes a hydrogen atom, if

$n^*$ denotes 3,

and their salts, characterised in that

a) an oxocarboxylic acid ester of the general formula II*

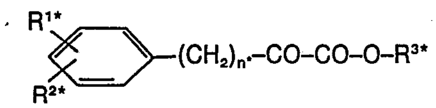

(II*)

wherein

$R^{1*}$, $R^{2*}$ and $n^*$ have the abovementioned meaning and

$R^{3*}$ denotes a lower alkyl radical,

is solvolysed and, if appropriate, resulting acids are subsequently converted into the salts or resulting salts are subsequently converted into the acids, or

b) a diester of the general formula III*

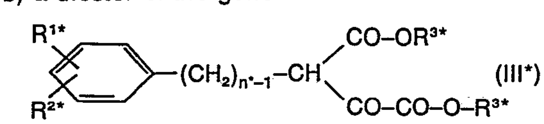

(III*)

wherein

$R^{1*}$, $R^{2*}$ and $n^*$ have the abovementioned meaning and

$R^{3*}$ denotes a lower alkyl radical,
is solvolysed and decarboxylated, and, if appropriate, resulting acids are subsequently converted into the salts or resulting salts are subsequently converted into the acids.

4. Process according to Claim 1 for the preparation of substituted oxocarboxylic acids of the general formula I**

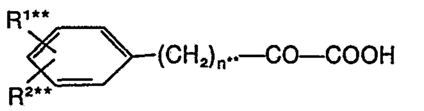 (I**)

wherein

$R^{1**}$ and $R^{2**}$ are in the meta-position or para-position to the ketocarboxylic acid radical, and

$R^{1**}$ denotes a hydrogen atom or a chlorine atom,

$R^{2**}$ denotes a chlorine atom and

n** denotes 3 to 5,

and their salts, characterised in that

a) an oxocarboxylic acid ester of the general formula II**

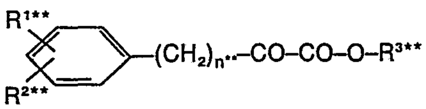 (II**)

wherein

$R^{1**}$, $R^{2**}$ and n** have the abovementioned meaning and

$R^{3**}$ denotes a methyl or ethyl radical,
is solvolysed and, if appropriate, resulting acids are subsequently converted into the salts or resulting salts are subsequently converted into the acids, or

b) a diester fo the general formula III**

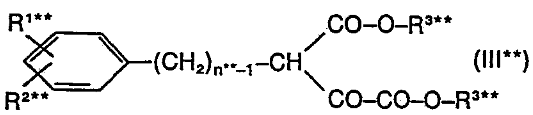 (III**)

wherein

$R^{1**}$, $R^{2**}$ and n** have the abovementioned meaning and

$R^{3**}$ denotes a methyl or ethyl radical,
is solvolysed and decarboxylated, and, if appropriate, resulting acids are subsequently converted into the salts or resulting salts are subsequently converted into the acids.

5. Process for the preparation of new medicaments which contain as active ingredient substituted oxocarboxylic acids of the general formula I′

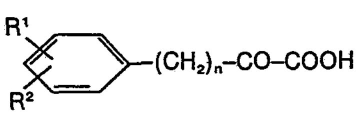 (I′)

wherein

$R^1$ denotes a hydrogen atom, a halogen atom, a hydroxyl group, a lower alkyl group, a lower alkoxy group or a trifluoromethyl group,

$R^2$ denotes a hydrogen atom or a halogen atom and

n denotes an integer from 3 to 8,

and/or their pharmacologically acceptable salts with inorganic or organic bases, characterised in that the active ingredient is mixed with a pharmaceutical carrier and – as the case may be – with a further additive.

6. Process according to claim 1, characterised in that the medicament is produced in unit dose form containing 2 to 200 mg of active substance.

7. Process according to claims 5 or 6, characterised in that the active substance is produced by a process according to one of the claims 1 to 4.

**Revendications pour les Etats contractants: BE, CH, DE, FR, GB, IT, LI, LU, NL, SE**

1. Acides oxocarboxyliques substitués de formule générale I:

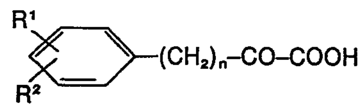 (I),

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8, étant entendu que $R^1$ ne représente pas ni un atome d'hydrogène, ni un atome de fluor, ni un groupe méthyle, ni un groupe méthoxyle et $R^2$ ne représente pas un atome d'hydrogène quand n a la signification 3, et leurs sels.

2. Composés selon la revendication 1, dans lesquels n représente un nombre entier de 3 à 6, $R^1$ ne représentant pas un atome d'hydrogène, un atome de fluor, un groupe méthyle ni un groupe méthoxyle et $R^2$ ne représentant pas un atome d'hydrogène quand n a la signification 3.

3. Acides oxocarboxyliques substitués selon la revendication 1, caractérisés par la formule générale I*:

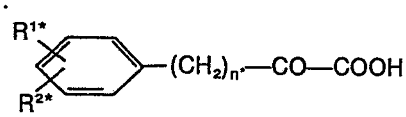 (I*)

dans laquelle:

$R^{1*}$ représente un atome d'hydrogène, un atome de chlore, un groupe méthyle, un groupe méthoxyle ou un groupe trifluorométhyle,

$R^{2*}$ représente un atome d'hydrogène ou un atome de chlore et

n* est un nombre entier de 3 à 6, $R^{1*}$ ne représentant pas un atome d'hydrogène, un groupe méthyle ni un groupe méthoxyle et $R^{2*}$ ne représentant pas un atome d'hydrogène quand n* est égal à 3, et leurs sels.

4. Acides oxocarboxyliques substitués selon la revendication 1, caractérisés par la formule générale I**:

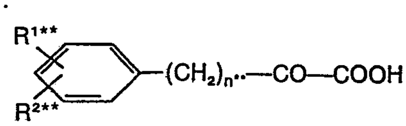 (I**)

dans laquelle $R^{1**}$ et $R^{2**}$ sont en position méta ou para relativement au radical acide cétocarboxylique et $R^{1**}$ représente un atome d'hydrogène ou un atome de chlore, $R^{2**}$ un atome de chlore et $n^{**}$ un nombre d 3 à 5,
et leurs sels pharmacologiquement tolérés de bases inorganiques.

5. Acides oxocarboxyliques substitués selon la revendication 1, caractérisés par la formule générale I':

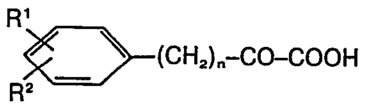 (I')

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8,
et leurs sels pharmacologiquement tolérés de bases inorganiques ou organiques, pour l'application dans le traitement de maladies qui reposent sur des perturbations du métabolisme du glucose.

6. Acice 5-(4-méthoxyphényl)-2-oxovalérique et ses sels pharmacologiquement tolérés de bases inorganiques ou organiques, pour l'utilisation dans le traitement de maladies qui reposent sur des perturbations du métabolisme du glucose.

7. Médicaments qui contiennent un ou plusieurs des acides oxocarboxyliques substitués de formule générale I':

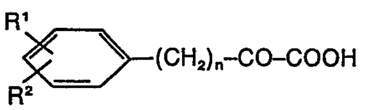 (I')

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8,
et/ou leurs sels pharmacologiquement tolérés de bases inorganiques ou organiques.

8. Médicaments selon la revendication 7, contenant des acides oxocarboxyliques substitués de formule générale I', dans laquelle $R^1$ représente un atome d'hydrogène un atome de chlore, un groupe méthyle, ou groupe méthoxyle ou un groupe trifluorométhyle, $R^2$ un atome d'hydrogène ou un atome de chlore et n un nombre entier de 3 à 6, et/ou leurs sels pharmacologiquement tolérés.

9. Médicaments selon la revendication 7, contenant des composés selon la revendication 3.

10. Médicaments selon la revendication 7, contenant des composés selon la revendication 4.

11. Procédé pour la préparation des acides oxocarboxyliques substitués de formule générale I:

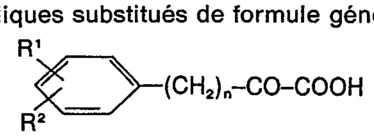 (I)

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8, étant entendu que $R^1$ ne représente pas ni un atome d'hydrogène, ni un atome de fluor, ni un groupe méthyle, ni un groupe méthoxyle et $R^2$ ne représente pas un atome d'hydrogène quand n a la signification 3, et de leurs sels, caractérisés par le fait:

a) que l'on solvolyse un ester d'acide oxocarboxylique de formule générale II:

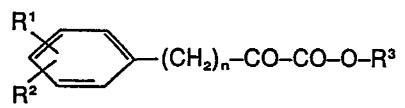 (II)

dans laquelle $R^1$, $R^2$ et n ont la signification indiquée ci-dessus et $R^3$ représente un radical alkyle inférieur et qu'éventuellement, on convertit ensuite des acides obtenus en sels ou des sels obtenus en acides, ou

b) que l'on solvolyse un diester de formule générale III:

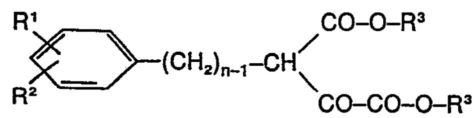 (III)

dans laquelle $R^1$, $R^2$, $R^3$ et n ont la signification indiquée ci-dessus et qu'on le décarboxyle, et qu'éventuellement on convertit ensuite des acides obtenus en sels ou des sels obtenus en acides.

12. Procédé selon la revendication 11, caractérisé en ce que l'on utilise comme composés de départ des esters II ou diesters III d'acide oxocarboxylique dans lesquels n représente un nombre entier de 3 à 6, $R^1$ ne représentant pas un atome d'hydrogène, un atome de fluor, un groupe méthyle ni un groupe méthoxyle et $R^2$ ne représentant pas un atome d'hydrogène quand n a la signification 3.

13. Procédé selon la revendication 11, pour la préparation d'acides oxocarboxyliques substitués de formule générale I*:

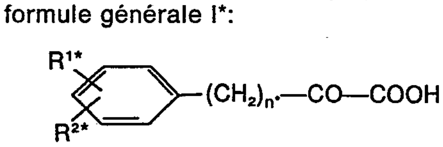 (I*)

dans laquelle:

$R^{1*}$ représente un atome d'hydrogène, un atome de chlore, un groupe méthyle, un groupe méthoxyle ou un groupe trifluorométhyle,

$R^{2*}$ représente un atome d'hydrogène ou un atome de chlore et

$n^*$ est un nombre entier de 3 à 6, $R^{1*}$ ne représentant pas un atome d'hydrogène, un groupe méthyle ni un groupe méthoxyle et $R^{2*}$ ne représentant pas un atome d'hydrogène quand $n^*$ est égal à 3,
et de leurs sels, caractérisé par le fait:

a) que l'on solvolyse un ester d'acide oxocarboxylique de formule générale II*:

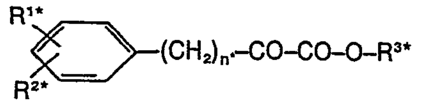
(II*)

dans laquelle $R^{1*}$, $R^{2*}$ et n* ont la signification indiquée ci-dessus et $R^{3*}$ représente un radical alkyle inférieur et qu'éventuellement, on convertit ensuite des acides obtenus en sels ou des sels obtenus en acides, ou

b) que l'on solvolyse un diester de formule générale III*:

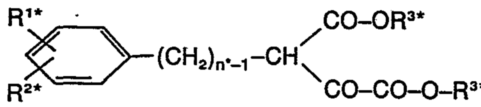
(III*)

dans laquelle $R^{1*}$, $R^{2*}$ et n* ont la signification indiquée ci-dessus et $R^{3*}$ représente un radical alkyle inférieur et qu'on le décarboxyle, et qu'éventuellement, on convertit ensuite des acides obtenus en sels ou des sels obtenus en acides.

14. Procédé selon la revendication 1, pour la préparation d'acides oxocarboxyliques substitués de formule générale I**:

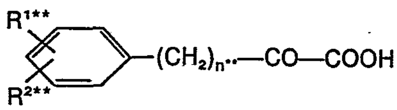
(I**)

dans laquelle $R^{1**}$ et $R^{2**}$ sont en position méta ou para relativement au radical acide cétocarboxylique et $R^{1**}$ représente un atome d'hydrogène ou un atome de chlore, $R^{2**}$ un atome de chlore et n** un nombre de 3 à 5, et de leurs sels, caractérisé par le fait:

a) que l'on solvolyse un ester d'acide oxocarboxylique de formule générale II**:

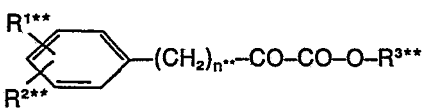
(II**)

dans laquelle $R^{1**}$, $R^{2**}$ et n** ont la signification indiquée ci-dessus et $R^{3**}$ représente un radical méthyle ou éthyle et qu'éventuellement, on convertit ensuite des acides obtenus en sels ou des sels obtenus en acides, ou

b) que l'on solvolyse un diester de formule générale III**:

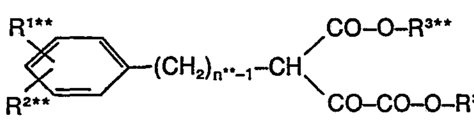
(III**)

dans laquelle $R^{1**}$, $R^{2**}$ et n** ont la signification indiquée ci-dessus et $R^{3**}$ représente un radical méthyle ou éthyle et qu'on le décarboxyle, et qu'éventuellement on convertit ensuite des acides obtenus en sels ou des sels obtenus en acides.

**Revendications l'Etat contractant: AT**

1. Procédé pour la préparation des acides oxocarboxyliques substitués de formule générale I:

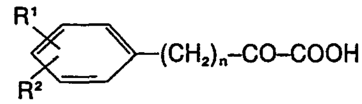
(I),

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8, étant entendu que $R^1$ ne représente pas ni un atome d'hydrogène, ni un atome de fluor, ni un groupe méthyle, ni un groupe méthoxyle et $R^2$ ne représente pas un atome d'hydrogène quand n a la signification 3, et de leurs sels, caractérisés par le fait:

a) que l'on solvolyse un ester d'acide oxocarboxylique de formule générale II:

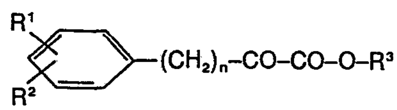
(II)

dans laquelle $R^1$, $R^2$ et n ont la signification indiquée ci-dessus et $R^3$ représente un radical alkyle inférieur et qu'éventuellement, on convertit ensuite des acides obtenus en sels ou des sels obtenus en acides, ou

b) que l'on solvolyse un diester de formule générale III:

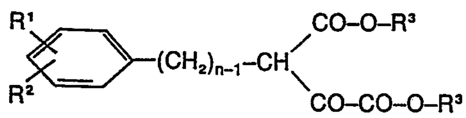
(III)

dans laquelle $R^1$, $R^2$, $R^3$ et n ont la signification indiquée ci-dessus et qu'on décarboxyle, et qu'éventuellement on convertit ensuite des acides obtenus en sels ou des sels obtenus en acide.

2. Procédé selon la revendication 1, caractérisé par le fait que l'on utilise comme composés de départ des esters II ou diesters III d'acide oxocarboxylique dans lesquels n représente un nombre entier de 3 à 6, $R^1$ ne représentant pas un atome d'hydrogène, un atome de fluor, un groupe méthyle ni un groupe méthoxyle et $R^2$ ne représentant pas un atome d'hydrogène quand n a la signification 3.

3. Procédé selon la revendication 1, pour la préparation d'acides oxocarboxyliques substitués de formule générale I*:

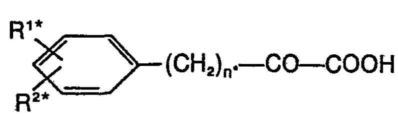
(I*)

dans laquelle:

$R^{1*}$ représente un atome d'hydrogène, un atome de chlore, un groupe méthyle, un groupe méthoxyle ou un groupe trifluorométhyle,

$R^{2*}$ représente un atome d'hydrogène ou un atome de chlore et

n* est un nombre entier de 3 à 6, $R^{1*}$ ne représentant pas un atome d'hydrogène, un groupe méthyle ni un groupe méthoxyle et $R^{2*}$ ne représentant pas un atome d'hydrogène quand n est égal à 3,

et de leurs sels, caractérisé par le fait:

a) que l'on solvolyse un ester d'acide oxocarboxylique de formule générale II*:

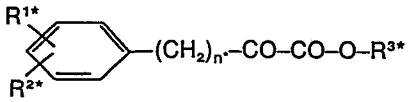
(II*)

dans laquelle $R^{1*}$, $R^{2*}$ et n* ont la signification indiquée ci-dessus et $R^{3*}$ représente un radical alkyle inférieur et qu'éventuellement, on convertit ensuite des acides obtenus en sels ou des sels obtenus en acides, ou

b) que l'on solvolyse un diester de formule générale III*:

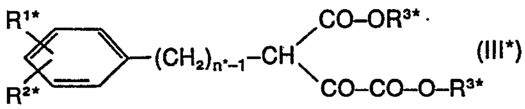
(III*)

dans laquelle $R^{1*}$, $R^{2*}$ et n* ont la signification indiquée ci-dessus et $R^{3*}$ représente un radical alkyle inférieur et qu'on le décarboxyle, et qu'éventuellement, on convertit ensuite des acides obtenus en sels ou des sels obtenus en acides.

4. Procédé selon la revendication 1, pour la préparation d'acides oxocarboxyliques substitués de formule générale I**:

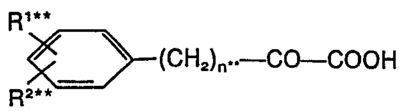
(I**)

dans laquelle $R^{1**}$ et $R^{2**}$ sont en position méta ou para relativement au radical acide cétocarboxylique et $R^{1**}$ représente un atome d'hydrogène ou un atome de chlore, $R^{2**}$ un atome de chlore et n** un nombre de 3 à 5,

et de leurs sels, caractérisé par le fait:

a) que l'on solvolyse un ester d'acide oxocarboxylique de formule générale II**:

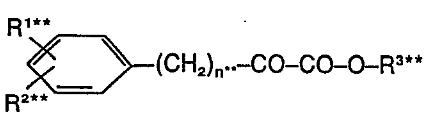
(II**)

dans laquelle $R^{1**}$, $R^{2**}$ et n** ont la signification indiquée ci-dessus et $R^{3**}$ représente un radical méthyle ou éthyle et qu'éventuellement, on convertit ensuite des acides obtenus en sels ou des sels obtenus en acides, ou

b) que l'on solvolyse un diester de formule générale III**:

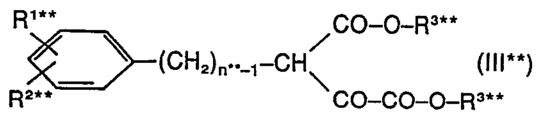
(III**)

dans laquelle $R^{1**}$, $R^{2**}$ et n** ont la signification indiquée ci-dessus et $R^{**}$ représente un radical méthyle ou éthyle et qu'on le décarboxyle, et qu'éventuellement on convertit ensuite des acides obtenus en sels ou des sels obtenus en acides.

5. Procédé de préparation de médicaments nouveaux qui contiennent comme substance active des acides oxocarboxyliques substitués de formule générale I':

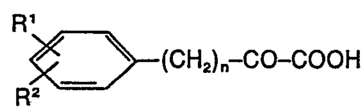
(I')

dans laquelle:

$R^1$ représente un atome d'hydrogène, un atome d'halogène, un groupe hydroxyle, un groupe alkyle inférieur, un groupe alcoxyle inférieur ou un groupe trifluorométhyle,

$R^2$ représente un atome d'hydrogène ou un atome d'halogène et

n est un nombre entier de 3 à 8,

et/ou leurs sels pharmacologiquement tolérés de bases inorganiques ou organiques, caractérisé par le fait que l'on mélange la substance active à un véhicule pharmaceutique et éventuellement à d'autres additifs.

6. Procédé selon la revendication 5, caractérisé par le fait que l'on prépare le médicament sous la forme d'une dose unité qui contient 2 à 200 mg de substance active.

7. Procédé selon l'une des revendications 5 et 6, caractérisé par le fait que l'on a préparé la substance active à l'aide d'un procédé selon l'une des revendications 1 à 4.